# EUROPEAN PATENT SPECIFICATION

(11) **EP 3 371 327 B1**
(45) Date of publication and mention of the grant of the patent: **18.12.2024**
(21) Application number: 16862785.9
(22) Date of filing: 01.11.2016
(51) Int. Cl.: C12Q 1/68, G01N 33/574, G01N 33/53, C12Q 1/6806, C12Q 1/6886, C12Q 1/6841

(54) **SINGLE CELL GENOMIC PROFILING OF CIRCULATING TUMOR CELLS (CTCS) IN METASTATIC DISEASE TO CHARACTERIZE DISEASE HETEROGENEITY**
GENOMISCHE EINZELZELLENPROFILIERUNG VON ZIRKULIERENDEN TUMORZELLEN (CTCS) BEI METASTASISCHER ERKRANKUNG ZUR CHARAKTERISIERUNG DER ERKRANKUNGSHETEROGENITÄT
PROFILAGE GÉNOMIQUE MONOCELLULAIRE DE CELLULES TUMORALES CIRCULANTES (CTC) DANS UNE MALADIE MÉTASTATIQUE PERMETTANT DE CARACTÉRISER L'HÉTÉROGÉNÉITÉ DE LA MALADIE

(30) Priority: 03.11.2015 US 201562250422 P
(43) Date of publication of application: 12.09.2018
(73) Proprietor: Epic Sciences, Inc., San Diego, CA 92121 (US)
(72) Inventor: DITTAMORE, Ryan, San Diego, CA 92121 (US); MARRINUCCI, Dena, San Diego, CA 92121 (US)
(74) Representative: Jones Day
(86) International application number: PCT/US2016/059877
(87) International publication number: WO 2017/079139

(56) References cited:
- WO-A1-2015/048740
- WO-A1-2015/112955
- WO-A2-2014/165785
- US-A1- 2015 147 339
- US-A1- 2015 212 089
- US-A1- 2015 233 927
- SHANNON L. WERNER ET AL: "Analytical Validation and Capabilities of the Epic CTC Platform: Enrichment-Free Circulating Tumour Cell Detection and Characterization", JOURNAL OF CIRCULATING BIOMARKERS, vol. 4, no. 3, 5 May 2015 (2015-05-05), pages 1 - 13, XP055282360, DOI: 10.5772/60725
- PAUL MEDVEDEV ET AL: "Computational methods for discovering structural variation with next-generation sequencing", NATURE METHODS, vol. 6, no. 11s, 1 November 2009 (2009-11-01), pages S13 - S20, XP055065779, ISSN: 1548-7091, DOI: 10.1038/nmeth.1374
- SHAOJUN ZHANG ET AL: "A Genomic Instability Score in Discriminating Nonequivalent Outcomes of BRCA1/2 Mutations and in Predicting Outcomes of Ovarian Cancer Treated with Platinum-Based Chemotherapy", PLOS ONE, vol. 9, no. 12, 1 December 2014 (2014-12-01), pages e113169, XP055418698, DOI: 10.1371/journal.pone.0113169
- GAETANO ZAFARANA ET AL: "Copy number alterations of c-MYC and PTEN are prognostic factors for relapse after prostate cancer radiotherapy", CANCER., vol. 118, no. 16, 26 January 2012 (2012-01-26), US, pages 4053 - 4062, XP055401642, ISSN: 0008-543X, DOI: 10.1002/cncr.26729
- RATHKOPF ET AL.: "Androgen Receptor Antagonists in Castration-Resistant Prostate Cancer.", CANCER J., vol. 19, no. 1, 1 January 2013 (2013-01-01), pages 43 - 49, XP055541514, DOI: 10.1097/PPO.0b013e318282635a
- CHANG: "Treatment Options for Hormone-Refractory Prostate Cancer.", REV UROL., vol. 9, no. Suppl 2, 2007, pages 13 - 8, XP 055382652
- SHAFFER ET AL.: "Circulating Tumor Cell Analysis in Patients with Progressive Castration-Resistant Prostate Cancer.", CLIN CANCER RES., vol. 13, no. 7, 2007, pages 2023 - 9, XP 055064896

## Description

The invention relates generally to the field of cancer diagnostics and, more specifically to methods for single cell genomic profiling of circulating tumor cells (CTCs) to characterize disease heterogeneity in a cancer patient.

### BACKGROUND

After successive cancer therapies, multiple subpopulations of cancer cells arise, each with divergent genetic aberrations that may confer drug resistance or susceptibility. Tissue biopsies may not detect these subpopulations, but a liquid biopsy of blood can help identify these important tumor cells and characterize how a patient's tumors have evolved over time. Single cell genomic profiling is a powerful new tool for investigating evolution and diversity in cancer and understanding the role of rare cells in tumor progression. Clonal diversity is destined to play an important role in invasion, metastasis, and the evolution of resistance to therapy.

Prostate cancer is the most commonly diagnosed solid organ malignancy in the United States (US) and remains the second leading cause of cancer deaths among American men. In 2014 alone, the projected incidence of prostate cancer is 233,000 cases with deaths occurring in 29,480 men, making metastatic prostate cancer therapy truly an unmet medical need. Siegel et al., 2014. CA Cancer J Clin. 2014;64(1):9-29. Epidemiological studies from Europe show comparable data with an estimated incidence of 416700 new cases in 2012, representing 22.8% of cancer diagnoses in men. In total, 92200 PC-specific deaths are expected, making it one of the three cancers men are most likely to die from, with a mortality rate of 9.5%

Despite the proven success of hormonal therapy for prostate cancer using chemical or surgical castration, most patients eventually will progress to a phase of the disease that is metastatic and shows resistance to further hormonal manipulation. This has been termed metastatic castration-resistant prostate cancer (mCRPC). Despite this designation, however, there is evidence that androgen receptor (AR)-mediated signaling and gene expression can persist in mCRPC, even in the face of castrate levels of androgen. This may be due in part to the upregulation of enzymes involved in androgen synthesis, the overexpression of AR, or the emergence of mutant ARs with promiscuous recognition of various steroidal ligands. Androgen receptor (AR)-gene amplification, found in 20-30% of mCRPC is proposed to develop as a consequence of hormone-deprivation therapy and be a prime cause of treatment failure. Treatment of patients with mCRPC remains a significant clinical challenge. Studies have further elucidated a direct connection between the PI3K-AKT-mTOR and androgen receptor (AR) signaling axes, revealing a dynamic interplay between these pathways during the development of hormone resistance. PTEN is one of the most commonly deleted/mutated tumor suppressorgenes in human prostate cancer. As a lipid phosphatase and negative regulator of the PI3K/AKT/mTOR pathway, PTEN controls a number of cellular processes, including survival, growth, proliferation, metabolism, migration, and cellular architecture. PTEN loss can be used as a diagnostic and prognostic biomarker for prostate cancer, as well as predict patient responses to emerging PI3K/AKT/mTOR inhibitors.

Prior to 2004, there was no treatment proven to improve survival for men with mCRPC. The treatment of patients with mitoxantrone with prednisone or hydrocortisone was aimed only at alleviating pain and improving quality of life, but there was no benefit in terms of overall survival (OS). In 2004, the results of two major phase 3 clinical trials, TAX 327 and SWOG (Southwest Oncology Group) 9916, established Taxotere^{®} (docetaxel) as a primary chemotherapeutic option for patients with mCRPC. Additional hormonal treatment with androgen receptor (AR) targeted therapies, chemotherapy, combination therapies, and immunotherapy, has been investigated for mCRPC, and recent results have offered additional options in this difficult-to-treat patient group. With the advent of exponential growth of novel agents tested and approved for the treatment of patients with metastatic castration-resistant prostate cancer (mCRPC) in the last 5 years alone, issues regarding the optimal sequencing or combination of these agents have arisen. Several guidelines exist that help direct clinicians as to the best sequencing approach and most would evaluate presence or lack of symptoms, performance status, as well as burden of disease to help determine the best sequencing for these agents. Mohler et al., 2014, J Natl Compr Canc Netw. 2013;11(12):1471-1479; Cookson et al., 2013, J Urol. 2013;190(2):429-438. Currently, approved treatments consist of taxane-class cytotoxic agents such as Taxotere^{®} (docetaxel) and Jevtana^{®} (cabazitaxel), and anti-androgen hormonal therapy drugs such as Zytiga^{®} (arbiterone, blocks androgen production) or Xtandi^{®} (enzalutamide, an androgen receptor (AR) inhibitor).

The challenge for clinicians is to decide the best sequence for administering these therapies to provide the greatest benefit to patients. However, therapy failure remains a significant challenge based on heterogeneous responses to therapies across patients and in light of cross-resistance from each agent. Mezynski et al., Ann Oncol. 2012;23(11):2943-2947; Noonan et al., Ann Oncol. 2013;24(7): 1802-1807; Pezaro et al., Eur Urol. 2014, 66( 3): 459-465. In addition, patients may lose the therapeutic window to gain substantial benefit from each drug that has been proven to provide overall survival gains. Hence, better methods of identifying the target populations who have the most potential to benefit from targeted therapies remain an important goal.

Circulating tumor cells (CTCs) represent a significant advance in cancer diagnosis made even more attractive by their non-invasive measurement. Cristofanilli et al., N Engl J Med 2004, 351:781-91. CTCs released from either a primary tumor or its metastatic sites hold important information about the biology of the tumor. Historically, the extremely low levels of CTCs in the bloodstream combined with their unknown phenotype has significantly impeded their detection and limited their clinical utility. A variety of technologies have recently emerged for detection, isolation and characterization of CTCs in order to utilize their information. CTCs have the potential to provide a non-invasive means of assessing progressive cancers in real time during therapy, and further, to help direct therapy by monitoring phenotypic physiological and genetic changes that occur in response to therapy. In most advanced prostate cancer patients, the primary tumor has been removed, and CTCs are expected to consist of cells shed from metastases, providing a "liquid biopsy." While CTCs are traditionally defined as EpCAM/cytokeratin positive (CK+) cells, CD45-, and morphologically distinct, recent evidence suggests that other populations of CTC candidates exist including cells that are EpCAM/cytokeratin negative (CK-) or cells smaller in size than traditional CTCs. These findings regarding the heterogeneity of the CTC population, suggest that enrichment-free CTC platforms are favorable over positive selection techniques that isolate CTCs based on size, density, or EpCAM positivity that are prone to miss important CTC subpopulations. Shannon L. W. et al. Journal of Circulating Biomarkers, vol. 4, no. 3, 5 May 2015 (2015-05-05), pages 1-13 and WO 2015/048740 relate to analytical validation and capabilities of the Epic CTC platform applied to enrichment-free detection and characterization (cell markers, morphological parameters, copy number variation (CNV) etc.) of circulating tumor cells. WO 2014/165785 describes the use of high throughput sequencing data to analyse CNV and genome instability, e.g., large-scale transitions (LSTs). Zhang S. et al. PLOS ONE, vol. 9, no. 12, 1 December 2014 (2014-12-01), page e113169 discloses the provision of genomic instability score based on CNV and number of mutations.

CRPC presents serious challenges to both the patients suffering from this advanced form of PrCa and the clinicians managing these patients. Clinicians are often faced with providing comprehensive diagnoses and assessments of the mechanisms that cause disease progression in an effort to guide appropriate and individualized treatments. By identifying appropriate therapeutic and prognostic markers, the potential clinical benefit of targeted therapy is increased, and clinicians are enabled to better managed CRPC, improve the quality of life for patients, and enhance clinical outcomes. A need exists to understand the frequency of subclonal CNV driver alterations and genomic instability in individual CTCs in combination with cell phenotype to enable a more accurate view of heterogeneous disease, predict therapeutic response, and identify novel mechanisms of resistance. The present invention addresses this need and provides related advantages are provided.

### SUMMARY OF THE INVENTION

The present invention provides a method of detecting heterogeneity of disease in a cancer patient comprising (a) performing a direct analysis comprising immunofluorescent staining and morphological characteristization of nucleated cells in a blood sample obtained from the patient to identify and enumerate circulating tumor cells (CTC); (b) isolating the CTCs from the sample; (c) individually characterizing genomic parameters to generate a genomic profile for each of the CTCs; and (d) determining heterogeneity of disease in the cancer patient based on the profile, wherein said genomic parameters comprise genomic instability characterized by measuring large scale transitions (LSTs) and percentage of a patient's genome harboring copy number alterations (PGA). In some embodiments, the cancer is prostate cancer. In some embodiments, the prostate cancer hormone refractory.

In some embodiments, the immunofluorescent staining of nucleated cells comprises pan cytokeratin, cluster of differentiation (CD) 45, diamidino-2-phenylindole and (DAPI).

In some embodiments, the genomic parameters comprise copy number variation (CNV) signatures. In some embodiments, the CNV signatures comprise gene amplifications or deletions. In some embodiments, the gene amplifications comprise amplification of AR gene. In some embodiments, the deletions comprise loss of Phosphatase and tensin homolog gene (PTEN). In some embodiments, the CNV signatures comprise genes associated with androgen independent cell growth.

According to the invention, the genomic parameters comprise genomic instability. Specifically, the genomic instability is characterized by measuring large scale transitions (LSTs) and percent genome altered (PGA).

Other features and advantages of the invention will be apparent from the detailed description.

### BRIEF DESCRIPTION OF THE DRAWINGS

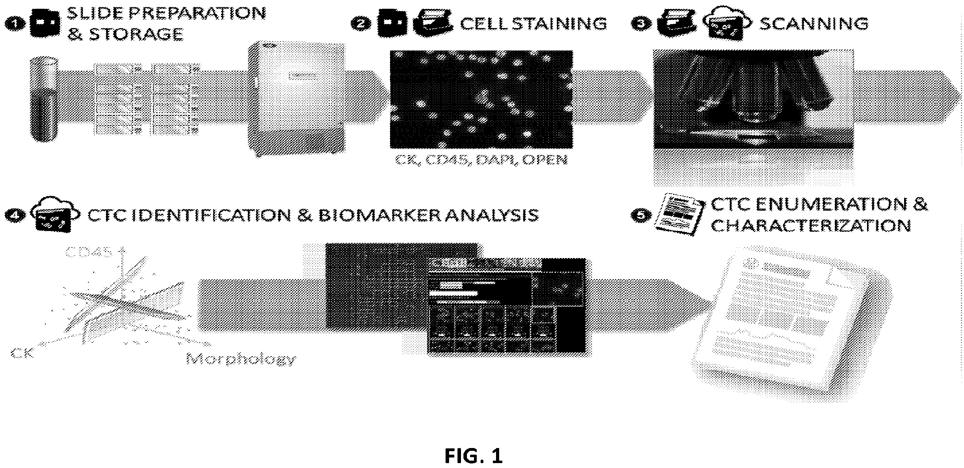

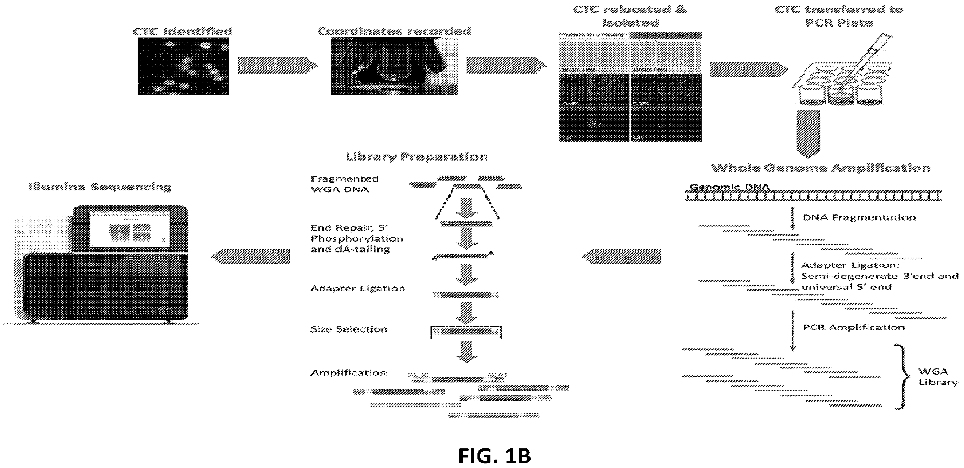

Figure 1A shows a description of standard Epic CTC analysis process. Images are analyzed using a multi-parametric digital pathology algorithm to detect CTC candidates and quantitate protein biomarker expression levels. CTC classifications are displayed in a web-based report and are confirmed by trained technicians. Figure 1B shows a description of the CTC recovery and genomic profiling workflow. Individual cells are isolated, subjected to Whole Genome Amplification, and NGS library preparation. Sequencing is performed on an Illumina NextSeq 500.

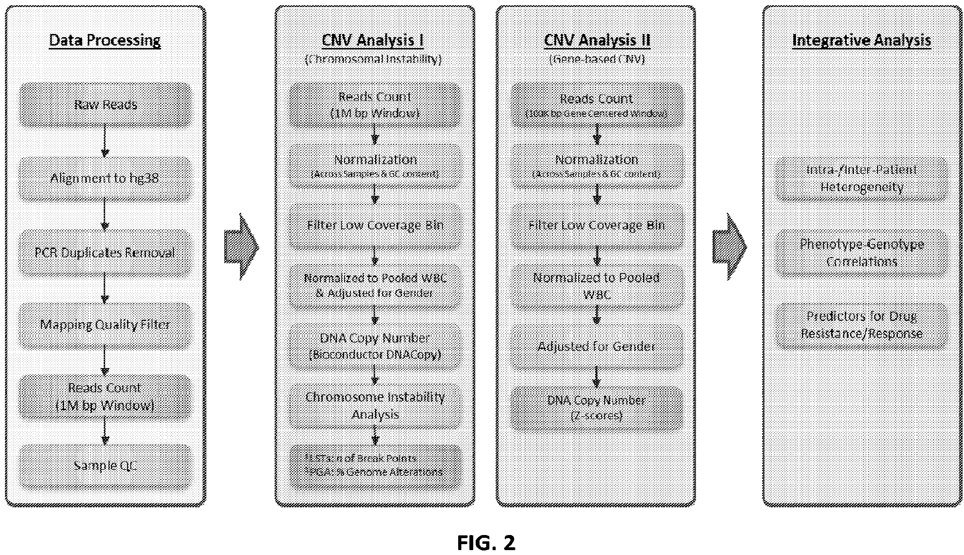

Figure 2 provides a diagram of the bioinformatic analysis performed. Raw FASTQ files are assessed and filtered for quality. Reads are aligned to the hg 38 reference genome (UCSC), PCR duplicates removed, and filtered by the MAPQ score 30. Samples with >250K reads post filtering are analyzed for copy number alterations. The filtered alignment files are further analyzed with Epic's Copy Number Pipelines. One pipeline was for estimating genomic instability using 1M bp window, and the other was for gene specific copy number measurement. ¹ LSTs: n of chromosomal breaks between adjacent regions of at least 10 Mb. ² PGAs: percentage of a patient's genome harboring copy number alterations (amplification or deletions).

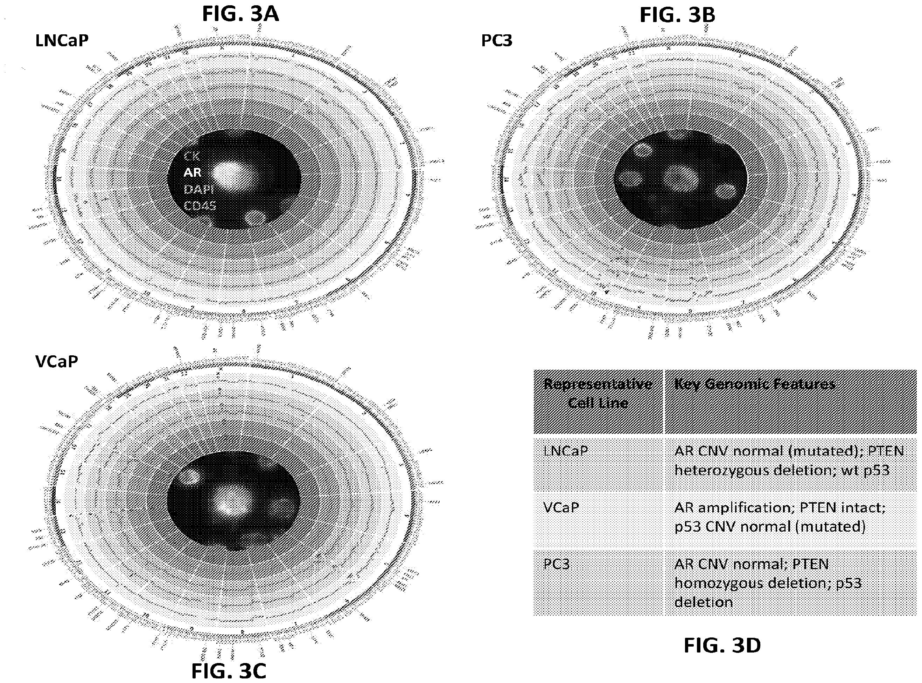

Figures 3A-3C show copy number variations (CNVs) in single cells. Single cells each from LNCaP, PC3, and VCaP were isolated and analyzed by whole genome sequencing for copy number variations. Amplifications and deletions can be observed reproducibly across replicates. Representative images of each cell line are also shown. Cells are stained with a CK cocktail, AR, CD45, and DAPI. Replicates of 5 from each cell line are shown here to demonstrate reproducibility. Known genomic alterations from each cell line are described in Figure 3D. Plots were generated with Circos: Krzywinski, M. et al. Circos: an Information Aesthetic for Comparative Genomics. Genome Res (2009) 19:1639-1645

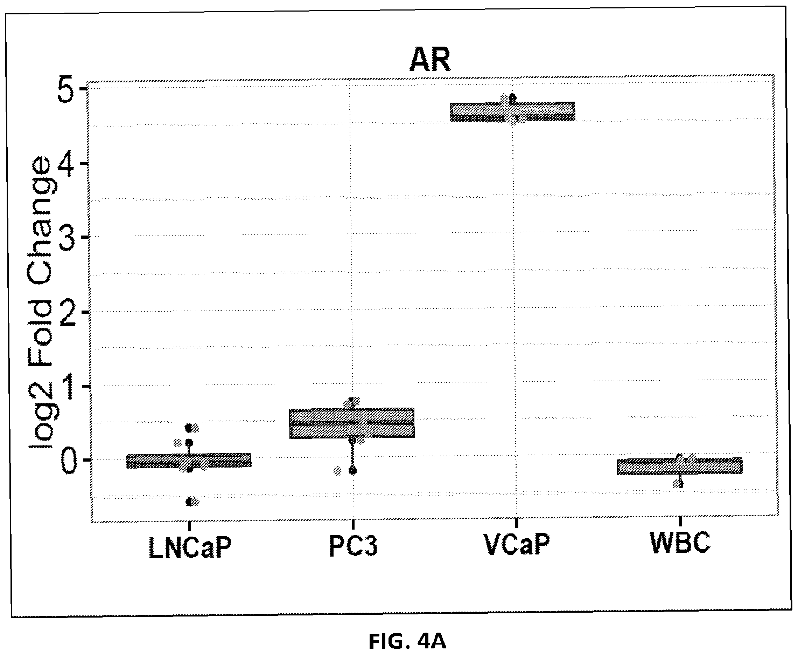

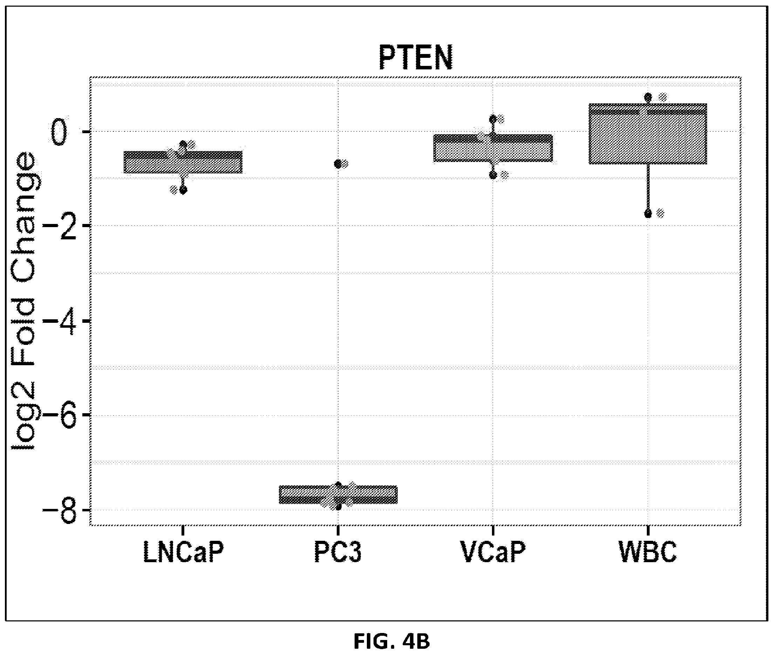

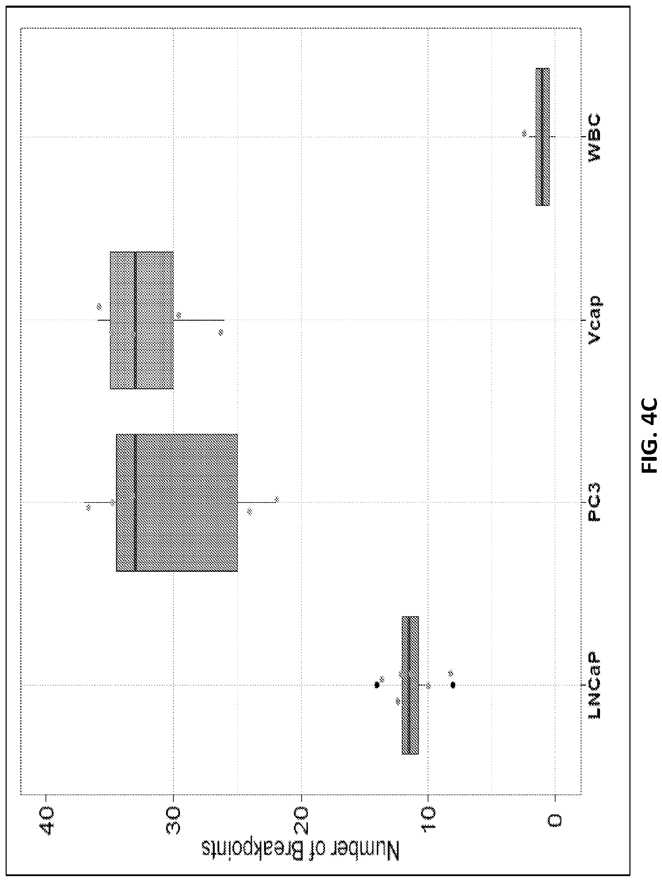

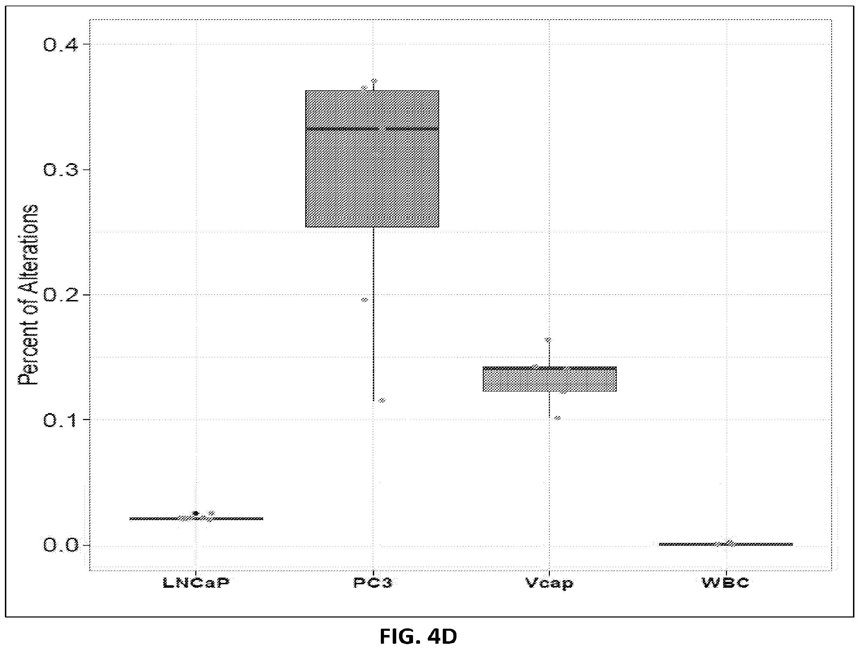

Figures 4A-4D show CNV (Figures 4A and 4B) and Genomic Instability Measurements (Figures 4C and 4D). Figure 4A shows comparison of log2 genomic copy number of AR in 3 representative cell lines and healthy donor white blood cell (WBC) control. VCaP harbors an amplification of AR, while LNCaP and PC3 maintain 2 copies of AR. Figure 4B shows comparison of log2 genomic copy number of PTEN in 3 representative cell lines and healthy donor WBC control. PC3 homozygous PTEN loss was confirmed, LNCaP heterozygous PTEN loss was observed in many cells with significant z-scores. Figure 4C shows comparison of the # of breakpoints (LSTs) across 3 representative cell line and healthy donor WBC control. A higher number of breakpoints were detected in PC3 (PTEN null, p53 mutant) and VCaP (p53 mutant) in comparison to LNCaPs (wt p53 and heterozygous PTEN loss) and the WBC control. Figure 4D shows comparison of the % of genome altered in 3 representative cell lines and healthy donor WBC control. PC3 displayed the highest percent of alterations, revealing genetic instability and polyploidy, likely due to loss of both PTEN and p53.

### DETAILED DESCRIPTION

The present disclosure is based, in part, on the discovery that integrated single cell whole genome CNV analysis provides reproducible copy number profiles across multiple replicates and confirms the presence of known focal CNV events including AR amplification and PTEN loss. The present disclosure is further based, in part, on the discovery that hole genome copy number analysis can be used to reproducibly characterize genomic instability by measuring LSTs and PGA. As disclosed herein, the highest genomic instability detected in p53 mutant cell lines (PC3 & VCaP) compared to wild-type (LNCaP). Understanding the frequency of subclonal CNV driver alterations and genomic instability in individual CTCs in combination with cell phenotype may enable a more accurate view of heterogeneous disease, potential therapeutic response, and identify novel mechanisms of resistance.

Increased intra-tumor heterogeneity has been correlated with intrinsic resistance to therapy and poor outcome. CTCs have been shown to reflect heterogeneous disease and the active metastatic tumor population in metastatic patients. The non-enrichment CTC analysis platform described herein enables the methods of the invention by allowing for single cell resolution and accurate genomic profiling of heterogeneous CTC populations. To characterize intra-tumor heterogeneity single cell whole genome copy number analysis of circulating tumor cells (CTCs) was performed using a non-enrichment CTC analysis platform. Markers of therapeutic sensitivity, such as PTEN deletion or androgen receptor (AR) amplification for PI3K inhibitors or AR-targeted therapy, respectively, were detected in individual prostate cancer cells spiked into blood to mimic patient samples. In addition to the detection of focal actionable alterations, genomic instability was characterized by measuring large scale transitions (LSTs) and % genome altered (PGA).

The present invention provides a method of detecting heterogeneity of disease in a cancer patient comprising (a) performing a direct analysis comprising immunofluorescent staining and morphological characteristization of nucleated cells in a blood sample obtained from the patient to identify and enumerate circulating tumor cells (CTC); (b) isolating the CTCs from the sample; (c) individually characterizing genomic parameters to generate a genomic profile for each of the CTCs, wherein said genomic parameters comprise genomic instability characterized by measuring large scale transitions (LSTs) and percentage of a patient's genome harboring copy number alterations (PGA); and (d) determining heterogeneity of disease in the cancer patient based on the profile. In some embodiments, the cancer is prostate cancer. In some embodiments, the prostate cancer is hormone refractory.

In some embodiments, the immunofluorescent staining of nucleated cells comprises pan cytokeratin, cluster of differentiation (CD) 45, diamidino-2-phenylindole (DAPI) and androgen receptor (AR).

In some embodiments, the genomic parameters comprise copy number variation (CNV) signatures. In some embodiments, the CNV signatures comprise gene amplifications or deletions. In some embodiments, the gene amplifications comprise amplification of AR gene. In some embodiments, the deletions comprise loss of Phosphatase and tensin homolog gene (PTEN). In some embodiments, the CNV signatures comprise genes associated with androgen independent cell growth.

According to the invention, the genomic parameters comprise genomic instability. Specifically, the genomic instability is characterized by measuring large scale transitions (LSTs) and percent genome altered (PGA).

In some embodiments, determining heterogeneity of disease in the cancer patient based on the profile identifies novel mechanisms of disease.

In some embodiments, determining heterogeneity of disease in the cancer patient based on the profile predicts a positive response to a treatment.

In some embodiments, determining heterogeneity of disease in the cancer patient based on the profile predicts a resistance to a treatment.

It must be noted that, as used in this specification and the appended claims, the singular forms "a", "an" and "the" include plural referents unless the content clearly dictates otherwise. Thus, for example, reference to "a biomarker" includes a mixture of two or more biomarkers, and the like.

The term "about," particularly in reference to a given quantity, is meant to encompass deviations of plus or minus five percent.

As used in this application, including the appended claims, the singular forms "a," "an," and "the" include plural references, unless the content clearly dictates otherwise, and are used interchangeably with "at least one" and "one or more."

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements does not include only those elements but can include other elements not expressly listed or inherent to such process, method, product-by-process, or composition of matter.

As used herein, the term "providing" used in the context of a liquid biopsy sample is meant to encompass any and all means of obtaining the sample. The term encompasses all direct and indirect means that result in presence of the sample in the context of practicing the claimed methods.

The term "patient," as used herein preferably refers to a human, but also encompasses other mammals. It is noted that, as used herein, the terms "organism," "individual," "subject," or "patient" are used as synonyms and interchangeably.

As used in the compositions and methods described herein, the term "cancer" refers to or describes the physiological condition in mammals that is typically characterized by unregulated cell growth. In one embodiment, the cancer is an epithelial cancer. In one embodiment, the cancer is prostate cancer. In various embodiments of the methods and compositions described herein, the cancer can include, without limitation, breast cancer, lung cancer, prostate cancer, colorectal cancer, brain cancer, esophageal cancer, stomach cancer, bladder cancer, pancreatic cancer, cervical cancer, head and neck cancer, ovarian cancer, melanoma, and multidrug resistant cancer, or subtypes and stages thereof. In still an alternative embodiment, the cancer is an "early stage" cancer. In still another embodiment, the cancer is a "late stage" cancer. The term "tumor," as used herein, refers to all neoplastic cell growth and proliferation, whether malignant or benign, and all pre-cancerous and cancerous cells and tissues. The cancer can be a lymphoproliferative cancer, for example, a precursor B lymphoblastic leukemia/lymphoblastic lymphoma, a B cell non-Hodgkin lymphomas of follicular origin, a Hodgkin lymphoma precursor T cell lymphoblastic leukemia/lymphoblastic lymphoma, a neoplasm of immature T cells, a neoplasm of peripheral, post-thymic T cells, a T cell prolymphocytic leukemia, a peripheral T cell lymphoma, an unspecified, anaplastic large cell lymphoma, an adult T cell leukemia/lymphoma, a chronic lymphocytic leukemia, a mantle cell lymphoma, a follicular lymphoma, a marginal zone lymphoma, a hairy cell leukemia, a diffuse large B cell lymphoma, a Burkitt lymphoma, a lymphoplasmacytic lymphoma, a precursor T lymphoblastic leukemia/lymphoblastic lymphoma, a T cell prolymphocytic leukemia, an angioimmunoblastic lymphoma, or a nodular lymphocyte predominant Hodgkin lymphoma.

As used herein, the term "circulating tumor cell" or "CTC" is meant to encompass any rare cell that is present in a biological sample and that is related to cancer. CTCs, which can be present as single cells or in clusters of CTCs, are often epithelial cells shed from solid tumors found in very low concentrations in the circulation of patients.

As used herein, a "traditional CTC" refers to a single CTC that is cytokeratin positive, CD45 negative, contains a DAPI nucleus, and is morphologically distinct from surrounding white blood cells.

As used herein, a "non-traditional CTC" refers to a CTC that differs from a traditional CTC in at least one characteristic.

In its broadest sense, a biological sample can be any sample that contains CTCs. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains cells and encompasses any material in which CTCs can be detected. A sample can be, for example, whole blood, plasma, saliva or other bodily fluid or tissue that contains cells.

According to the invention, the biological sample is a blood sample. As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulations that are distinguishable by methods well known in the art (*e.g.,* FACS, immunohistochemistry). For example, a blood sample can contain populations of non-nucleated cells, such as erythrocytes (*e.g.,* 4-5 million/µl) or platelets (150,000-400,000 cells/µl), and populations of nucleated cells such as WBCs (*e.g*., 4,500 - 10,000 cells/µl), CECs or CTCs (circulating tumor cells; *e.g.,* 2-800 cells/ µl). WBCs may contain cellular subpopulations of, *e.g.,* neutrophils (2,500-8,000 cells/µl), lymphocytes (1,000-4,000 cells/µl), monocytes (100-700 cells/µl), eosinophils (50-500 cells/µl), basophils (25 - 100 cells/ µl) and the like. The samples of this disclosure are non-enriched samples, *i.e.,* they are not enriched for any specific population or subpopulation of nucleated cells. For example, non-enriched blood samples are not enriched for CTCs, WBC, B-cells, T-cells, NK-cells, monocytes, or the like.

A blood sample may be obtained from a healthy subject or a subject deemed to be at high risk for cancer or metastasis of existing cancer based on art known clinically established criteria including, for example, age, race, family snd history. According to the invention, the blood sample is obtained from a cancer patient. In some embodiments the blood sample is from a subject who has been diagnosed with cancer based on tissue or liquid biopsy and/or surgery or clinical grounds. In some embodiments, the blood sample is obtained from a subject showing a clinical manifestation of cancer and/or well known in the art or who presents with any of the known risk factors for a particular cancer. In some embodiments, the cancer is bladder cancer, for example, urothelial bladder cancer.

As used herein in the context of generating CTC data, the term direct analysis means that the CTCs are detected in the context of all surrounding nucleated cells present in the sample as opposed to after enrichment of the sample for CTCs prior to detection. In some embodiments, the methods comprise microscopy providing a field of view that includes both CTCs and at least 200 surrounding white blood cells (WBCs).

A fundamental aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables characterization of CTC, including subtypes of CTCs described herein, that allows for identification of phenotypes and heterogeneity that cannot be achievied with other CTC detection methods and that enables the analysis of biomarkers in the context of the claimed methods.

In some embodiments, the methods disclosed herein can further take account of individual patient risk factors and imaging data, which includes any form of imaging modality known and used in the art, for example and without limitation, by X-ray computed tomography (CT), ultrasound, positron emission tomography (PET), electrical impedance tomography and magnetic resonance (MRI). It is understood that one skilled in the art can select an imaging modality based on a variety of art known criteria. As described herein, the methods of the invention can encompass one or more pieces of imaging data. In the methods disclosed herein, one or more individual risk factors can be selected from the group consisting of age, race, family history. It is understood that one skilled in the art can select additional individual risk factors based on a variety of art known criteria. As described herein, the methods of the invention can encompass one or more individual risk factors. Accordingly, biomarkers can include imaging data, individual risk factors and CTC data. As described herein, biomarkers also can include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (*e.g.,* glycoproteins, ribonucleoproteins, lipoproteins) as well as portions or fragments of a biological molecule.

CTC data can include morphological, genetic, epigenetic features and immunofluorescent features. As will be understood by those skilled in the art, biomarkers can include a biological molecule, or a fragment of a biological molecule, the change and/or the detection of which can be correlated, individually or combined with other measurable features, with cancer. CTCs, which can be present a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1: 1,000 in a blood cell population, *e.g.,* an abundance of less than 1:5,000, 1: 10,000, 1:30,000, 1:50:000, 1: 100,000, 1:300,000, 1:500,000, or 1: 1,000,000. In some embodiments, the a CTC has an abundance of 1:50:000 to 1:100,000 in the cell population.

The samples of this disclosure may be obtained by any means, including, *e.g.,* by means of solid tissue biopsy or fluid biopsy (*see, e.g.,* Marrinucci D. *et al.,* 2012, Phys. Biol. 9 016003). Briefly, in particular embodiments, the process can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (*e.g.,* procedures for drawing and processing whole blood). In some embodiments, a blood sample is drawn into anti-coagulent blood collection tubes (BCT), which may contain EDTA or Streck Cell-Free DNA^{™}. In other embodiments, a blood sample is drawn into CellSave^{®} tubes (Veridex). A blood sample may further be stored for up to 12 hours, 24 hours, 36 hours, 48 hours, or 60 hours before further processing.

In some embodiments, the methods of this disclosure comprise an intitial step of obtaining a white blood cell (WBC) count for the blood sample. In certain embodiments, the WBC count may be obtained by using a HemoCue^{®} WBC device (Hemocue, Ängelholm, Sweden). In some embodiments, the WBC count is used to determine the amount of blood required to plate a consistent loading volume of nucleated cells per slide and to calculate back the equivalent of CTCs per blood volume.

In some embodiments, the methods of this disclosure comprise an initial step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e.g.,* in a PBS solution.

In some embodiments, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 µm. In some embodiments, the material is a film. In some embodiments the material is a glass slide. In certain embodiments, the method encompasses an initial step of depositing nucleated cells from the blood sample as a monolayer on a glass slide. The glass slide can be coated to allow maximal retention of live cells (*See, e.g.,* Marrinucci D. *et al.,* 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto the glass slide. In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI). In some embodiments, immunofluorescent staining of nucleated cells comprises pan cytokeratin (CK), cluster of differentiation (CD) 45 and DAPI. In some embodiments further described herein, CTCs comprise distinct immunofluorescent staining from surrounding nucleated cells. In some embodiments, the distinct immunofluorescent staining of CTCs comprises DAPI (+), CK (+) and CD 45 (-). In some embbodiments, the identification of CTCs further comprises comparing the intensity of pan cytokeratin fluorescent staining to surrounding nucleated cells. In some embodiments, the CTC data is generated by fluorescent scanning microscopy to detect immunofluorescent staining of nucleated cells in a blood sample. Marrinucci D. et al., 2012, Phys. Biol. 9 016003.

In particular embodiments, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified as DAPI (+), CK (+) and CD 45 (-). In the methods described herein, the CTCs comprise distinct immunofluorescent staining from surrounding nucleated cells.

In further embodiments, the CTC data includes traditional CTCs also known as high definition CTCs (HD-CTCs). Traditional CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during HD-CTC enumeration as previously described. Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous.

While CTCs can be identified as comprises DAPI (+), CK (+) and CD 45 (-) cells, the methods of the invention can be practiced with any other biomarkers that one of skill in the art selects for generating CTC data and/or identifying CTCs and CTC clusters. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide

A person skilled in the art will appreciate that a number of methods can be used to generate CTC data, including microscopy based approaches, including fluorescence scanning microscopy (*see, e.g.,* Marrinucci D. et al., 2012, Phys. Biol. 9 016003), sequencing approaches, mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzyme-linked immunosorbant assay (ELISA), immunopercipitation, radioimmunoassay, dot blotting, and FACS. Immunoassay techniques and protocols are generally known to those skilled in the art ( Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and non-competitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

Standard molecular biology techniques known in the art and not specifically described are generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Md. (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998). Polymerase chain reaction (PCR) can be carried out generally as in PCR Protocols: A Guide to Methods and Applications, Academic Press, San Diego, Calif. (1990). Any method capable of determining a DNA copy number profile of a particular sample can be used for molecular profiling according to the invention provided the resolution is sufficient to identify the biomarkers of the invention. The skilled artisan is aware of and capable of using a number of different platforms for assessing whole genome copy number changes at a resolution sufficient to identify the copy number of the one or more biomarkers of the invention.

*In situ* hybridization assays are well known and are generally described in Angerer et al., Methods Enzymol. 152:649-660 (1987). In an *in situ* hybridization assay, cells, e.g., from a biopsy, are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters. FISH (fluorescence in situ hybridization) uses fluorescent probes that bind to only those parts of a sequence with which they show a high degree of sequence similarity.

FISH is a cytogenetic technique used to detect and localize specific polynucleotide sequences in cells. For example, FISH can be used to detect DNA sequences on chromosomes. FISH can also be used to detect and localize specific RNAs, e.g., mRNAs, within tissue samples. In FISH uses fluorescent probes that bind to specific nucleotide sequences to which they show a high degree of sequence similarity. Fluorescence microscopy can be used to find out whether and where the fluorescent probes are bound. In addition to detecting specific nucleotide sequences, e.g., translocations, fusion, breaks, duplications and other chromosomal abnormalities, FISH can help define the spatial-temporal patterns of specific gene copy number and/or gene expression within cells and tissues.

Nucleic acid sequencing technologies are suitable methods for analysis of gene expression. The principle underlying these methods is that the number of times a cDNA sequence is detected in a sample is directly related to the relative expression of the RNA corresponding to that sequence. These methods are sometimes referred to by the term Digital Gene Expression (DGE) to reflect the discrete numeric property of the resulting data. Early methods applying this principle were Serial Analysis of Gene Expression (SAGE) and Massively Parallel Signature Sequencing (MPSS). See, e.g., S. Brenner, et al., Nature Biotechnology 18(6):630-634 (2000). More recently, the advent of "next-generation" sequencing technologies has made DGE simpler, higher throughput, and more affordable. As a result, more laboratories are able to utilize DGE to screen the expression of more genes in more individual patient samples than previously possible. See, e.g., J. Marioni, Genome Research 18(9): 1509-1517 (2008); R. Morin, Genome Research 18(4):610-621 (2008); A. Mortazavi, Nature Methods 5(7):621-628 (2008); N. Cloonan, Nature Methods 5(7):613-619 (2008).

A person of skill in the art will futher appreciate that the presence or absence of biomarkers may be detected using any class of marker-specific binding reagents known in the art, including, e.g., antibodies, aptamers, fusion proteins, such as fusion proteins including protein receptor or protein ligand components, or biomarker-specific small molecule binders. In some embodiments, the presence or absence of CK or CD45 is determined by an antibody. The skilled person will further appreciate that the presence or absence of biomarkers can be measured by evaluating a chromosome copy number change at a chromosome locus of a biomarker. Genomic biomarkers can be identified by any technique such as, for example, comparative genomic hybridization (CGH), or by single nucleotide polymorphism arrays (genotyping microarrays) of cell lines, such as cancer cells. A bioinformatics approach can identify regions of chromosomal aberrations that discriminate between cell line groups and that are indicative of the biomarker, using appropriate copy number thresholds for amplifications and deletions in addition to further analysis using techniques such as qPCR or in situ hybridization. Nucleic acid assay methods for detection of chromosomal DNA copy number changes include: (i) in situ hybridization assays to intact tissue or cellular samples, (ii) microarray hybridization assays to chromosomal DNA extracted from a tissue sample, and (iii) polymerase chain reaction (PCR) or other amplification assays to chromosomal DNA extracted from a tissue sample. Assays using synthetic analogs of nucleic acids, such as peptide nucleic acids, in any of these formats can also be used.

The biomarker may be detected through hybridization assays using detectably labeled nucleic acid-based probes, such as deoxyribonucleic acid (DNA) probes or protein nucleic acid (PNA) probes, or unlabeled primers which are designed/selected to hybridize to the specific designed chromosomal target. The unlabeled primers are used in amplification assays, such as by polymerase chain reaction (PCR), in which after primer binding, a polymerase amplifies the target nucleic acid sequence for subsequent detection. The detection probes used in PCR or other amplification assays are preferably fluorescent, and still more preferably, detection probes useful in "real-time PCR". Fluorescent labels are also preferred for use in situ hybridization but other detectable labels commonly used in hybridization techniques, e.g., enzymatic, chromogenic and isotopic labels, can also be used. Useful probe labeling techniques are described in Molecular Cytogenetics: Protocols and Applications, Y - S. Fan, Ed., Chap. 2, "Labeling Fluorescence In Situ Hybridization Probes for Genomic Targets", L. Morrison et al., p. 21-40, Humana Press,.COPYRGT. 2002. In detection of the genomic biomarkers by microarray analysis, these probe labeling techniques are applied to label a chromosomal DNA extract from a patient sample, which is then hybridized to the microarray.

In other embodiments, a biomarker protein may be detected though immunological means or other protein assays. Protein assay methods useful in the invention to measure biomarker levels may comprise (i) immunoassay methods involving binding of a labeled antibody or protein to the expressed biomarker, (ii) mass spectrometry methods to determine expressed biomarker, and (iii) proteomic based or "protein chip" assays for the expressed biomarker. Useful immunoassay methods include both solution phase assays conducted using any format known in the art, such as, but not limited to, an ELISA format, a sandwich format, a competitive inhibition format (including both forward or reverse competitive inhibition assays) or a fluorescence polarization format, and solid phase assays such as immunohistochemistry (referred to as "IHC").

The antibodies for detecting a biomarker bind specifically to the biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some examples, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers when generating CTC data in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers in the methods of the invention. Suitable detectable labels include, but are not limited to, fluorescent dyes (*e.g.*, fluorescein, fluorescein isothiocyanate (FITC), Oregon Green^{™}, rhodamine, Texas red, tetrarhodimine isothiocynate (TRITC), Cy3, Cy5, Alexa Fluor^{®} 647, Alexa Fluor^{®} 555, Alexa Fluor^{®} 488), fluorescent markers (*e.g*., green fluorescent protein (GFP), phycoerythrin, etc.), enzymes (*e.g*., luciferase, horseradish peroxidase, alkaline phosphatase, etc.), nanoparticles, biotin, digoxigenin, metals, and the like.

For mass-sectrometry based analysis, differential tagging with isotopic reagents, *e.g.,* isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I; or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for epithelial cells In some embodiments, the immunofluorescent makers comprise a marker specific for white blood cells (WBCs). In some embodiments, one or more of the immunofluorescent markers comprise CD 45 and CK.

In some embodiments, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, CTCs comprise distinct morphological characteristics compared to surrounding nucleated cells. In some embodiments, the morphological characteristics comprise nucleus size, nucleus shape, cell size, cell shape, and/or nuclear to cytoplasmic ratio. In some embodiments, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC.

CTC data can be generated with any microscopic method known in the art. In some embodiments, the method is performed by fluorescent scanning microscopy. In certain embodiments the microscopic method provides high-resolution images of CTCs and their surrounding WBCs (*see, e.g.,* Marrinucci D. et al., 2012, Phys. Biol. 9 016003). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

In some embodiments, a CTC data includes detecting one or more biomarkers, for example, CK and CD 45. A biomarker is considered "present" in a cell if it is detectable above the background noise of the respective detection method used (*e.g.,* 2-fold, 3-fold, 5-fold, or 10-fold higher than the background; *e*.*g*., 2σ or 3σ over background). In some embodiments, a biomarker is considered "absent" if it is not detectable above the background noise of the detection method used (*e.g.,* <1.5-fold or <2.0-fold higher than the background signal; *e.g.,* <1.5σ or <2.0σ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (*i.e.,* the marker is considered present) if its fluorescent signal for the respective marker is significantly higher than the fixed background signal (*e.g.,* 2-fold, 3-fold, 5-fold, or 10-fold higher than the background; *e.g*., 2σ or 3σ over background). For example, a nucleated cell is considered CD 45 positive (CD 45⁺) if its fluorescent signal for CD 45 is significantly higher than the background signal. A cell is considered negative for an immunofluorescent marker (*i.e.*, the marker is considered absent) if the cell's fluorescence signal for the respective marker is not significantly above the background signal (*e.g.,* <1.5-fold or <2.0-fold higher than the background signal; *e.g.*, <1.5σ or <2.0σ over background).

Typically, each microscopic field contains both CTCs and WBCs. In certain embodiments, the microscopic field shows at least 1, 5, 10, 20, 50, or 100 CTCs. In certain embodiments, the microscopic field shows at least 10, 25, 50, 100, 250, 500, or 1,000 fold more WBCs than CTCs. In certain embodiments, the microscopic field comprises one or more CTCs or CTC clusters surrounded by at least 10, 50, 100, 150, 200, 250, 500, 1,000 or more WBCs.

In some embodiments of the methods described herein, generation of the CTC data comprises enumeration of CTCs that are present in the blood sample. In some embodiments, the methods described herein encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some embodiments of methods described herein, generation of the CTC data comprises detecting distinct subtypes of CTCs, including non-traditional CTCs. In some embodiments, the methods described herein encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular embodiment, the methods described herein encompass detection of at least 1 CTC cluster/mL of blood.

In some embodiments, the disclosed methods encompass the use of a predictive model. In further embodiments, the disclosed methods methods encompass comparing a measurable feature with a reference feature. As those skilled in the art can appreciate, such comparison can be a direct comparison to the reference feature or an indirect comparison where the reference feature has been incorporated into the predictive model. In further embodiments, analyzing a measurable encompasses one or more of a linear discriminant analysis model, a support vector machine classification algorithm, a recursive feature elimination model, a prediction analysis of microarray model, a logistic regression model, a CART algorithm, a flex tree algorithm, a LART algorithm, a random forest algorithm, a MART algorithm, a machine learning algorithm, a penalized regression method, or a combination thereof. In particular embodiments, the analysis comprises logistic regression. In additional embodiments, the determination is expressed as a risk score.

An analytic classification process can use any one of a variety of statistical analytic methods to manipulate the quantitative data and provide for classification of the sample. Examples of useful methods include linear discriminant analysis, recursive feature elimination, a prediction analysis of microarray, a logistic regression, a CART algorithm, a FlexTree algorithm, a LART algorithm, a random forest algorithm, a MART algorithm, machine learning algorithms and other methods known to those skilled in the art.

Classification can be made according to predictive modeling methods that set a threshold for determining the probability that a sample belongs to a given class. The probability preferably is at least 50%, or at least 60%, or at least 70%, or at least 80%, or at least 90% or higher. Classifications also can be made by determining whether a comparison between an obtained dataset and a reference dataset yields a statistically significant difference. If so, then the sample from which the dataset was obtained is classified as not belonging to the reference dataset class. Conversely, if such a comparison is not statistically significantly different from the reference dataset, then the sample from which the dataset was obtained is classified as belonging to the reference dataset class.

The predictive ability of a model can be evaluated according to its ability to provide a quality metric, *e.g*. AUROC (area under the ROC curve) or accuracy, of a particular value, or range of values. Area under the curve measures are useful for comparing the accuracy of a classifier across the complete data range. Classifiers with a greater AUC have a greater capacity to classify unknowns correctly between two groups of interest. ROC analysis can be used to select the optimal threshold under a variety of clinical circumstances, balancing the inherent tradeoffs that exist between specificity and sensitivity. In some embodiments, a desired quality threshold is a predictive model that will classify a sample with an accuracy of at least about 0.7, at least about 0.75, at least about 0.8, at least about 0.85, at least about 0.9, at least about 0.95, or higher. As an alternative measure, a desired quality threshold can refer to a predictive model that will classify a sample with an AUC of at least about 0.7, at least about 0.75, at least about 0.8, at least about 0.85, at least about 0.9, or higher.

As is known in the art, the relative sensitivity and specificity of a predictive model can be adjusted to favor either the specificity metric or the sensitivity metric, where the two metrics have an inverse relationship. The limits in a model as described above can be adjusted to provide a selected sensitivity or specificity level, depending on the particular requirements of the test being performed. One or both of sensitivity and specificity can be at least about 0.7, at least about 0.75, at least about 0.8, at least about 0.85, at least about 0.9, or higher.

The raw data can be initially analyzed by measuring the values for each measurable feature or biomarker, usually in triplicate or in multiple triplicates. The data can be manipulated, for example, raw data can be transformed using standard curves, and the average of triplicate measurements used to calculate the average and standard deviation for each patient. These values can be transformed before being used in the models, *e.g.* log-transformed, Box-Cox transformed (Box and Cox, Royal Stat. Soc., Series B, 26:211-246 (1964)). The data are then input into a predictive model, which will classify the sample according to the state. The resulting information can be communicated to a patient or health care provider. In some embodiments, the method has a specificity of >60%, >70%, >80%, >90% or higher.

As will be understood by those skilled in the art, an analytic classification process can use any one of a variety of statistical analytic methods to manipulate the quantitative data and provide for classification of the sample. Examples of useful methods include, without limitation, linear discriminant analysis, recursive feature elimination, a prediction analysis of microarray, a logistic regression, a CART algorithm, a FlexTree algorithm, a LART algorithm, a random forest algorithm, a MART algorithm, and machine learning algorithms.

The disclosure provides kits for the measurement of biomarker levels that comprise containers containing at least one labeled probe, protein, or antibody specific for binding to at least one of the expressed biomarkers in a sample. These kits may also include containers with other associated reagents for the assay. In some examples, a kit comprises containers containing a labeled monoclonal antibody or nucleic acid probe for binding to a biomarker and at least one calibrator composition. The kit can further comprise components necessary for detecting the detectable label (e.g., an enzyme or a substrate). The kit can also contain a control sample or a series of control samples which can be assayed and compared to the test sample. Each component of the kit can be enclosed within an individual container and all of the various containers can be within a single package, along with instructions for interpreting the results of the assays performed using the kit.

From the foregoing description, it will be apparent that variations and modifications can be made to the disclosure to adopt it to various usages and conditions.

The following examples are provided by way of illustration, not limitation.

### EXAMPLES

### Example 1.

Sample evaluation for CTCs was performed as reported previously using the Epic Sciences Platform. Marrinucci et al. Phys Biol 9:016003, 2012. The Epic CTC collection and detection process, which flows as follows: (1) Blood lysed, nucleated cells from blood sample placed onto slides; (2) Slides stored in -80C biorepository; (3) Slides stained with CK, CD45, DAPI and AR; (4) Slides scanned; (5) Multi-parametric digital pathology algorithms run, and (6) Software and human reader confirmation of CTCs & quantitation of biomarker expression. During the subsequent CTC recovery and genomic profiling workflow, individual cells were isolated, subjected to Whole Genome Amplification, and NGS library preparation. Sequencing was performed on an Illumina NextSeq 500.

Blood samples underwent hemolysis, centrifugation, re-suspension and plating onto slides, followed by -80⁰C storage. Prior to analysis, slides were thawed, labeled by immunofluorescence (pan cytokeratin, CD45, DAPI) and imaged by automated fluoroscopy then manual validation by a pathologist-trained technician (MSL). Marrinucci et al. Phys Biol 9:016003, 2012. DAPI (+), CK (+) and CD45 (-) intensities were categorized as features during CTC enumeration as previously described.

More specifically, peripheral blood sample was collected in Cell-free DNA BCT (Streck, Omaha, NE, USA) and shipped immediately to Epic Sciences (San Diego, CA, USA) at ambient temperature. Upon receipt, red blood cells were lysed and nucleated cells were dispensed onto glass microscope slides as previously described (Marrinucci et al. Hum Pathol 38(3): 514-519 (2007); Marrinucci et al. Arch Pathol Lab Med 133(9): 1468-1471 (2009); Mikolajczyk et al. J Oncol 2011: 252361. (2011); Marrinucci et al. Phys Biol 9(1): 016003 (2012); Werner et al. J Circ Biomark 4: 3 (2015)) and stored at -80 °C until staining. The millilitre equivalent of blood plated per slide was calculated based upon the sample's white blood cell count and the volume of post-RBC lysis cell suspension used. Circulating tumour cells were identified by immunofluorescence, as described (Marrinucci et al, 2007, *supra;* Marrinucci et al, 2009,*supra;* Mikolajczyk et al, 2011, *supra;* Marrinucci et al, 2012, *supra;* Werner et al, 2015, *supra).* During the subsequent CTC recovery and genomic profiling workflow, individual cells were isolated, subjected to Whole Genome Amplification, and NGS library preparation. Sequencing was performed on an Illumina NextSeq 500.

Figures 1 through 4 and the corresponding brief descriptions of the drawings describe further experimental details.

## Claims

1. A method of detecting heterogeneity of disease in a cancer patient comprising
(a) performing a direct analysis comprising immunofluorescent staining and morphological characterization of nucleated cells in a blood sample obtained from the patient to identify and enumerate circulating tumor cells (CTC);
(b) isolating the CTCs from said sample;
(c) individually characterizing genomic parameters to generate a genomic profile for each of the CTCs, and
(d) determining heterogeneity of disease in the cancer patient based on said profile,
wherein said genomic parameters comprise genomic instability **characterized by** measuring large scale transitions (LSTs) and percentage of a patient's genome harboring copy number alterations (PGA).

2. The method of claim 1, wherein said cancer is prostate cancer.

3. The method of claim 2, wherein said prostate cancer is hormone refractory.

4. The method of any one of claims 1-3, wherein the immunofluorescent staining of nucleated cells comprises pan cytokeratin, cluster of differentiation (CD) 45 and diamidino-2-phenylindole (DAPI).

5. The method of any one of claims 1-4, wherein said genomic parameters further comprise copy number variation (CNV) signatures.

6. The method of claim 5, wherein said copy number variation (CNV) signatures comprise gene amplifications or deletions.

7. The method of claim 6, wherein said CNV signatures comprise genes associated with androgen independent cell growth.

8. The method of claim 6, wherein said deletions comprise loss of phosphatase and tensin homolog (PTEN) gene.

9. The method of claim 6, wherein said gene amplifications comprise amplification of androgen receptor (AR) gene.

## Patentansprüche

1. Verfahren zum Erkennen von Heterogenität einer Erkrankung bei einem Krebspatienten, umfassend
(a) Durchführen einer direkten Analyse, umfassend Immunofluoreszenzfärbung und morphologische Charakterisierung von nukleierten Zellen in einer vom Patienten erhaltenen Blutprobe, um zirkulierende Tumorzellen (CTC) zu identifizieren und zählen;
(b) Isolieren der CTCs aus der Probe;
(c) individuelles Charakterisieren genomischer Parameter, um ein genomisches Profil für jede der CTCs zu erzeugen, und
(d) Bestimmen der Heterogenität der Erkrankung bei dem Krebspatienten auf Grundlage des Profils,
wobei die genomischen Parameter genomische Instabilität umfassen, die durch Messen von großskaligen Übergängen (LSTs) und eines Prozentsatzes des Patientengenoms, welches Kopienzahlveränderungen (PGA) aufweist, gekennzeichnet wird.

2. Verfahren nach Anspruch 1, wobei der Krebs Prostatakrebs ist.

3. Verfahren nach Anspruch 2, wobei der Prostatakrebs hormonrefraktär ist.

4. Verfahren nach einem der Ansprüche 1 bis 3, wobei die Immunofluoreszenzfärbung der nukleierten Zellen Pan-Zytokeratin, Differenzierungscluster (CD) 45 und Diamidino-2-phenylindol (DAPI) umfasst.

5. Verfahren nach einem der Ansprüche 1 bis 4, wobei die genomischen Parameter ferner Signaturen der Kopienzahlvariation (CNV) umfassen.

6. Verfahren nach Anspruch 5, wobei die Signaturen der Kopienzahlvariation (CNV) Genamplifikationen oder -deletionen umfassen.

7. Verfahren nach Anspruch 6, wobei die Signaturen der CNV Gene umfassen, die mit androgenunabhängigem Zellwachstum assoziiert sind.

8. Verfahren nach Anspruch 6, wobei die Deletionen Verlust des Phosphatase und Tensin-Homolog-Gens (PTEN) umfassen.

9. Verfahren nach Anspruch 6, wobei die Genamplifikationen Amplifikation des Androgenrezeptor (AR)-Gens umfassen.

## Revendications

1. Procédé de détection d'hétérogénéité de la maladie chez un patient atteint d'un cancer comprenant
(a) effectuer une analyse directe comprenant une coloration immunofluorescente et une caractérisation morphologique de cellules nucléées dans un échantillon de sang obtenu du patient pour identifier et énumérer des cellules tumorales circulantes (CTC) ;
(b) isoler les CTC dudit échantillon ;
(c) caractériser individuellement les paramètres génomiques pour générer un profil génomique pour chacune des CTC, et
(d) déterminer l'hétérogénéité de la maladie chez le patient cancéreux sur la base dudit profil,
dans lequel lesdits paramètres génomiques comprennent l'instabilité génomique **caractérisée par** la mesure de transitions à grande échelle (LST) et le pourcentage du génome d'un patient présentant des altérations du nombre de copies (PGA).

2. Procédé selon la revendication 1, dans lequel ledit cancer est un cancer de la prostate.

3. Procédé selon la revendication 2, dans lequel ledit cancer de la prostate est réfractaire aux hormones.

4. Procédé selon l'une quelconque des revendications 1 à 3, dans lequel la coloration immunofluorescente des cellules nucléées comprend la pan cytokératine, le cluster de différenciation (CD) 45 et le diamidino-2-phénylindole (DAPI).

5. Procédé selon l'une quelconque des revendications 1 à 4, dans lequel lesdits paramètres génomiques comprennent en outre des signatures de variation du nombre de copies (CNV).

6. Procédé selon la revendication 5, dans lequel lesdites signatures de variation du nombre de copies (CNV) comprennent des amplifications ou des suppressions de gènes.

7. Procédé selon la revendication 6, dans lequel lesdites signatures CNV comprennent des gènes associés à la croissance cellulaire indépendante des androgènes.

8. Procédé selon la revendication 6, dans lequel lesdites suppressions comprennent la perte du gène de l'homologue de la phosphatase et de la tensine (PTEN).

9. Procédé selon la revendication 6, dans lequel lesdites amplifications génétiques comprennent l'amplification du gène du récepteur des androgènes (AR).
